# FASCICULE DE BREVET EUROPEEN

(11) **EP 4 344 697 B1**
(45) Date de publication et mention de la délivrance du brevet: **02.07.2025**
(21) Numéro de dépôt: 22306443.7
(22) Date de dépôt: 28.09.2022
(51) Int. Cl.: A61K 8/85, A61Q 1/02

(54) **COMPOSITION COSMÉTIQUE COMPRENANT AU MOINS UN POLYHYDROXYALCANOATE COMPLEXE**
KOSMETISCHE ZUSAMMENSETZUNG MIT MINDESTENS EINEM KOMPLEXEN POLYHYDROXYALKANOAT
COSMETIC COMPOSITION COMPRISING AT LEAST ONE COMPLEX POLYHYDROXYALKANOATE

(43) Date de publication de la demande: 03.04.2024
(73) Titulaire: Chanel Parfums Beauté, 92200 Neuilly-sur-Seine (FR); Polymaris Biotechnology, 29200 Brest (FR)
(72) Inventeur: GUARILLOF, Philippe, 93500 PANTIN (FR); QUEVAUVILLER, Florence, 93500 PANTIN (FR); COURTOIS, Anthony, 29200 BREST (FR); THOLLAS, Bertrand, 29200 BREST (FR)
(74) Mandataire: Plasseraud IP

(56) Documents cités:
- WO-A1-99/35278
- CA-C- 1 335 370
- FR-A1- 3 111 557

## Description

### Domaine technique

La présente divulgation relève du domaine de la formulation cosmétique et plus particulièrement des compositions cosmétiques comprenant des biopolymères appartenant à la famille des polyhydroxyalcanoates, et conférant une persistance accrue d'au moins un effet cosmétique, notamment de maquillage et/ou de soin apporté après application de la composition, ainsi qu'une bonne adhésion de ladite composition après application sur les matières kératiniques.

De tels biopolymères permettent de conférer à ladite composition cosmétique des propriétés de longue tenue ainsi qu'une homogénéité à l'application, grâce à leur propriété filmogène.

### Technique antérieure

Les compositions cosmétiques de maquillage ou de soin sont couramment employées pour apporter un aspect esthétique lors de l'application sur la peau et les lèvres, cet effet devant perdurer au cours du temps. Elles doivent notamment résister aux différents facteurs extérieurs susceptibles de modifier leur effet esthétique, comme la sueur, les larmes, le sébum ou la salive. En particulier, dans le cas des rouges à lèvres, on cherche à avoir une bonne tenue dans le temps de la couleur et de la brillance, et une bonne résistance au transfert. Pour les fonds de teint, les fards à paupières et les poudres, on recherche également une bonne tenue dans le temps du maquillage et son non-transfert, tout en préservant la matité malgré l'apparition de sébum ou de sueur au cours de la journée. Enfin, pour les produits de protection solaire, la résistance au sébum et à la sueur sont également essentiels, de même que l'excellente homogénéité et la tenue du film déposé, lesquels influent significativement sur le niveau de protection aux ultra-violets obtenu.

Il est connu, afin de limiter le transfert de couleur des compositions cosmétiques et d'améliorer la tenue de leur teinte, d'incorporer dans les formules des polymères dits filmogènes. Les polymères filmogènes mis en oeuvre dans une composition cosmétique doivent être solubles dans la phase de la formule qui contient les ingrédients devant être fixés à la surface de la peau. Ils doivent également présenter une affinité pour la peau et favoriser l'adhérence du maquillage lors de l'application de la composition cosmétique sur la peau. Ils doivent aussi être agréables à appliquer et leur dépôt doit procurer une sensation de confort à l'utilisatrice, tout en conservant des propriétés esthétiques satisfaisantes. Enfin, ils doivent être facilement éliminés lors du nettoyage de la peau.

Il existe de nombreux polymères filmogènes commercialement disponibles, bien connus de l'homme de l'art et couramment utilisés dans le domaine de la cosmétique. A titre d'exemple illustratif, on peut notamment mentionner les dérivés de silicone tels que, par exemple, les résines MQ, par exemple les trimethylsiloxysilicates (ou résines T), les polysilsesquioxanes telles que le polypropylsilsesquioxane, ou bien encore les polymères dendrimères tels que l'acrylate/polytrimethylsiloxymethacrylate.

Toutefois, ces polymères filmogènes présentent un certain nombre d'inconvénients. En premier lieu, ils sont pour la plupart de nature synthétique et ne sont pas ou peu biodégradables. Il serait donc souhaitable de rechercher des solutions techniques plus respectueuses de l'environnement. De plus, les polymères de synthèse sont susceptibles de contenir des impuretés, telles que des unités monomériques n'ayant pas réagi, ou des traces de nanoparticules, les rendant incompatibles avec une utilisation cosmétique. Enfin, ils sont parfois difficiles à formuler et à stabiliser dans des compositions cosmétiques comprenant des ingrédients naturels tels que des extraits de plantes. Or, les ingrédients naturels sont de plus en plus utilisés dans les formulations cosmétiques, et peuvent par exemple être particulièrement intéressants pour apporter des fonctions d'hydratation ou d'anti-âge. FR3111557A1 divulgue une composition cosmétique comprenant au moins un copolymère de polyhydroxyalcanoate comprenant une répétition de cinq unités monomériques.

La présente invention vise donc à proposer une solution à ces différents problèmes, et à proposer la mise en oeuvre de nouveaux polymères filmogènes biocompatibles et biodégradables, dans des compositions cosmétiques.

### Description détaillée de l'invention

La présente invention concerne donc une composition cosmétique de maquillage et/ou de soin de la peau et/ou des lèvres, procurant un dépôt de maquillage ou de soin présentant une persistance dans le temps améliorée, permettant ainsi une meilleure tenue dans le temps de sa couleur et/ou de sa matité. Plus spécifiquement la présente divulgation concerne une composition cosmétique mettant en oeuvre, en tant qu'ingrédient filmogène, au moins un copolymère de polyhydroxyalcanoate, ledit copolymère étant issu de source biosourcée.

La présente invention a ainsi pour objet, selon un premier aspect, une composition cosmétique comprenant au moins un copolymère de polyhydroxyalcanoate, caractérisée en ce que ledit copolymère de polyhydroxyalcanoate consiste en une répétition d'unités monomériques de formules (A1), (B1), (C1), (D1), (E1), et (F1) suivantes :

Formule (A1) : -[-O-CH(R1)-(CH2)-CO-]-

Formule (B1) : -[-O-CH(R2)-(CH2)-CO-]-

Formule (C1) : -[-O-CH(R3)-(CH2)-CO-]-

Formule (D1) : -[-O-CH(R4)-(CH2)-CO-]-

Formule (E1) : -[-O-CH(R5)-(CH2)-CO-]-

Formule (F1) : -[-O-CH(R6)-(CH2)-CO-]-

dans lesquelles :
R1 représente un groupe n-propyle
R2 représente un groupe n-butyle
R3 représente un groupe n-pentyle
R4 représente un groupe n-hexyle
R5 représente un groupe n-heptyle
R6 représente un groupe n-octyle
ledit copolymère de polyhydroxyalcanoate comprenant au moins 1% en moles et au plus 50% en moles de chacune des d'unités monomériques de formule (A1), (B1), (C1), (D1), (E1), et (F1) .

Il est en effet du mérite de la demanderesse d'avoir mis en évidence que le choix d'unités polymériques (A1), (B1), (C1), (D1), (E1), et (F1) spécifiques, dans des proportions bien définies, permettait l'obtention de propriétés filmogènes particulièrement satisfaisantes, et améliorées par rapport à d'autres PHA connus.

La demanderesse a notamment mis en évidence que les polymères objets de la présente demande, consistant une répétition d'unités polymériques (A1), (B1), (C1), (D1), (E1), et (F1) spécifiques, dans des proportions bien définies, permettait l'obtention d'un composé aisément soluble dans divers solvants cosmétiques tel que l'isododécane. Les films obtenus sont stables dans le temps, et en particulier, résistent au sébum.

D'une façon préférée, le copolymère de polyhydroxyalcanoate consiste en :
- 1% à 3% en mole d'unités monomériques (A1),
- 2 à 9% en mole d'unités monomériques (B1),
- 20 à 30% en mole d'unités monomériques (C1)
- 10 à 30% en mole d'unités monomériques (D1)
- 25 à 50% en mole d'unités monomériques (E1)
- 9 à 20% en mole d'unités monomériques (F1).

D'une façon avantageuse, le copolymère de polyhydroxyalcanoate consiste en :
- 1% à 3% en mole d'unités monomériques (A1),
- 3 à 7% en mole d'unités monomériques (B1),
- 20 à 25% en mole d'unités monomériques (C1)
- 13 à 25% en mole d'unités monomériques (D1)
- 30 à 45% en mole d'unités monomériques (E1)
- 10 à 18% en mole d'unités monomériques (F1).

D'une façon particulièrement préférée, le copolymère de polyhydroxyalcanoate consiste en :
- 3% en mole d'unités monomériques (A1),
- 3% en mole d'unités monomériques (B1),
- 25% en mole d'unités monomériques (C1)
- 13% en mole d'unités monomériques (D1)
- 45% en mole d'unités monomériques (E1)
- 11% en mole d'unités monomériques (F1).

Un autre copolymère selon l'invention peut consister en :
- 2% en poids de poly(hydroxy-3-hexanoate),

6% en poids de poly(hydroxy-3-heptanoate),
21% en poids de poly(hydroxy-3-octanoate),
24% en poids de poly(hydroxy-3-nonanoate),
30% en poids de poly(hydroxy-3-decanoate), et
17% en poids de poly(hydroxy-3-undecanoate).

Encore un autre copolymère selon l'invention peut consister en :
- 1% en poids de poly(hydroxy-3-hexanoate),
- 5% en poids de poly(hydroxy-3-heptanoate),
- 22% en poids de poly(hydroxy-3-octanoate),
- 15% en poids de poly(hydroxy-3-nonanoate),
- 43% en poids de poly(hydroxy-3-decanoate), et
- 14% en poids de poly(hydroxy-3-undecanoate).

La composition cosmétique selon l'invention comprend au moins un copolymère de polyhydroxyalcanoate consistant en une répétition, successive ou non, d'unités monomériques (A1), (B1), (C1), (D1), (E1), et (F1) telles que décrites précédemment, et présentant un poids moléculaire moyen (Mw) compris entre 20 000 à 100 000 g/mol.

Le copolymère de polyhydroxyalcanoate est présent dans la composition cosmétique en une teneur comprise entre 0,5 % à 30% en poids, de préférence 1% à 20% en poids, et très préférentiellement 2% à 8% en poids, par rapport au poids total de la composition.

Selon un autre aspect, l'invention a pour objet l'utilisation d'au moins un copolymère de polyhydroxyalcanoate en tant qu'agent filmogène dans une composition cosmétique, ledit copolymère de polyhydroxyalcanoate consistant en une répétition d'unités monomérique de formules (A1), (B1), (C1), (D1), (E1), et (F1) suivantes :

Formule (A1) : -[-O-CH(R1)-(CH2)-CO-]-

Formule (B1) : -[-O-CH(R2)-(CH2)-CO-]-

Formule (C1) : -[-O-CH(R3)-(CH2)-CO-]-

Formule (D1) : -[-O-CH(R4)-(CH2)-CO-]-

Formule (E1) : -[-O-CH(R5)-(CH2)-CO-]-

Formule (F1) : -[-O-CH(R6)-(CH2)-CO-]-

dans lesquelles :
R1 représente un groupe n-propyle
R2 représente un groupe n-butyle
R3 représente un groupe n-pentyle
R4 représente un groupe n-hexyle
R5 représente un groupe n-heptyle
R6 représente un groupe n-octyle
ledit copolymère de polyhydroxyalcanoate comprenant au moins 1 % en moles et au plus 50% en moles de chacune des d'unités monomériques de formule (A1), (B1), (C1), (D1), (E1), et (F1) ;
ledit copolymère de polyhydroxyalcanoate étant obtenu par un procédé de fermentation à partir d'une bactérie enregistrée en vertu du traité de Budapest à la Collection Nationale de Cultures de Microorganismes le 24 février 2021 sous le numéro de dépôt I-5658 ; et étant présent en une teneur comprise entre 2 et 20% en poids, par rapport au poids total de ladite composition cosmétique.

Par « polymère filmogène », on entend, au sens de la présente demande, un polymère apte à former, à lui seul ou en présence d'un agent auxiliaire de filmification, un film macroscopiquement continu sur un support, notamment sur les matières kératiniques, et en particulier un film cohésif dont la cohésion et les propriétés mécaniques sont telles qu'il peut être isolé dudit support sans se déchirer.

La composition cosmétique selon l'invention peut se présenter sous la forme d'une émulsion, simple ou multiple et de type eau dans huile ou huile dans eau, d'une microémulsion, d'une nanoémulsion, d'une poudre, d'une composition anhydre, d'une suspension, d'une lotion, d'une crème, d'un gel aqueux ou hydroalcoolique, d'une mousse, d'un sérum, d'une solution ou d'une dispersion pour aérosol, ou d'une dispersion de vésicules lipidiques, ou de gouttelettes obtenues par coacervation ou par microfluidique.

Ladite composition cosmétique peut également comprendre, outre le copolymère de polyhydroxyalcanoate selon l'invention, au moins un additif usuel dans le domaine cosmétique, tel que par exemple au moins un composé choisi parmi un agent émollient ou humectant, un agent gélifiant et/ou épaississant, un agent tensioactif, une huile, une cire, un colorant, un pigment, une nacre, une charge telle que des agents de toucher, un conservateur, un agent antioxydant, un agent actif, une poudre organique ou inorganique, un solvant volatile et/ou non volatile, un solubilisant, un chélatant, un séquestrant, un sel ionique apte notamment à ajuster le pH de la composition ou sa force ionique, un filtre solaire pouvant être organique et/ou inorganique et un parfum. Selon la galénique souhaitée, le bénéfice soin et/ou maquillage recherché et attendu, la composition cosmétique peut également comprendre un autre polymère filmogène en plus du au moins un copolymère de polyhydroxyalcanoate selon l'invention, ledit autre filmogène étant préférentiellement respectueux de l'environnement, en particulier biodégradable.

Notamment, ladite composition peut contenir un ou plusieurs agent(s) émollient(s) ou humectant(s), qui peuvent être choisi(s) par exemple parmi la glycérine, les polyols, les glycols, les silicones.

S'il est présent dans la composition, ledit agent émollient ou humectant peut-être présent dans la composition à une teneur de l'ordre de 1 à 30%, de préférence 2 à 10% en poids, par rapport au poids total de la composition.

Un ou plusieurs agent(s) gélifiants(s) et/ou épaississant(s) de la phase aqueuse, peuvent également être incorporés à la composition selon l'invention. Ils sont choisis par exemple parmi les dérivés cellulosiques, les polysaccharides dont notamment les gommes d'origine végétale (guar, caroube, alginates, carraghénanes, pectine), d'origine microbienne (xanthane), les argiles (laponite), les gélifiants hydrophiles, et les gélifiants lipophiles.

Les gélifiants hydrophiles peuvent notamment être choisis parmi :
- les homo- ou copolymères d'acides acrylique ou méthacrylique ou leurs sels, les copolymères d'acide acrylique et d'acrylamide et leur sel de sodium, les sels de sodium d'acides polyhydroxycarboxyliques, les copolymères acide polyacryliques/acrylates d'alkyl, l'AMPS (Acide polyacrylamidométhyl propane sulfonique) et ses copolymères acrylamide ou de méthacrylates d'alkyle polyoxyéthylénés (réticulés ou non), et leurs mélanges.
- les polyuréthanes associatifs ou des solutions ou dispersions de ces polyuréthanes associatifs notamment dans l'eau ou en milieu hydroalcoolique.

Le terme « gélifiant lipophile », désigne, dans le cadre de la présente demande, une substance capable de solidifier ou de gélatiniser l'huile présente dans la composition de l'invention. Le gélifiant lipophile est choisi par exemple parmi les polyesters de saccharose. On peut citer les esters de saccharose et d'acides gras, et de préférence les esters de saccharose, de l'acide stéarique et de l'acide acétique, tels que le triacétate de tétrastéarate de saccharose.

S'ils sont présents dans la composition, les gélifiants peuvent être introduits en une teneur allant de 0,05 à 40% en poids, par rapport au poids total de la composition, de préférence de 0,1 à 20% et mieux de 0,5 à 15% en poids.

Un ou plusieurs agent(s) tensioactif(s), de préférence non ionique, peuvent faire partie de la composition selon l'invention et, s'ils sont présents dans la composition, seront présents dans une teneur de l'ordre de 0,1 à 8%, de préférence 0,5 à 3% en poids, par rapport au poids total de la composition.

Les compositions selon la présente demande peuvent aussi contenir au moins une ou plusieurs huiles ou solvant organique.

Par huile ou solvant organique, on entend un corps non aqueux liquide à température ambiante et pression atmosphérique. L'huile peut être volatile ou non volatile.

Par " huile ou solvant organique volatile", on entend au sens de l'invention tout milieu non aqueux susceptible de s'évaporer au contact des matières kératiniques en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant de 0,13 Pa à 40 000 Pa (10 3 à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

Par "huile non volatile", on entend une huile restant sur les matières kératiniques à température ambiante et pression atmosphérique au moins plusieurs heures et ayant notamment une pression de vapeur inférieure à 10⁻³ mm de Hg (0,13Pa).

L'huile peut être présente dans la composition dans une teneur allant de 0,05 à 30% de préférence 0,1 à 15% en poids par rapport au poids total de la composition. La composition selon l'invention peut comprendre des huiles volatiles et/ou des huiles non volatiles, et leurs mélanges.

Les huiles (ou solvants organiques) volatiles peuvent être des huiles hydrocarbonées, des huiles siliconées, linéaire(s), cyclique(s) ou ramifié(s), ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C₈-C₁₆ d'origine naturelle ou pétrolière, comme les isoalcanes en C₈-C₁₆ tels que l'isododécane (encore appelé 2,2,4,4,6-pentaméthylheptane), l'isodécane, l'isohexadécane, les esters ramifiés en C₈-C₁₆, le néopentanoate d'iso-hexyle, et leurs mélanges.

Comme huiles volatiles, on peut aussi utiliser les silicones volatiles, comme par exemple les huiles de silicones linéaires ou cycliques volatiles, notamment celles ayant une viscosité 6 centistokes (6.10 -6 m 2 /s), et ayant notamment de 3 à 6 atomes de silicium, ces silicones comportant éventuellement un ou plusieurs groupes alkyles ou alkoxy ayant de 1 ou 2 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

Chacune des compositions conformes à l'invention peut également comprendre au moins une huile ou solvant organique non volatile, qui peut être en particulier choisie parmi les huiles hydrocarbonées et/ou siliconées non volatiles.

Comme huile hydrocarbonée non volatile, on peut notamment citer :
- les huiles hydrocarbonées d'origine végétale telles que les triglycérides constitués d'esters d'acides gras et de glycérol dont les acides gras peuvent avoir des longueurs de chaînes variées de C4 à C24, ces derniers pouvant être linéaires ou ramifiés, saturés ou insaturés ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat, de camélia ; ou encore les triglycérides des acides caprylique/caprique;
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters naturels comme par exemple les triglycérides, ou les dérivés d'acide pélargoniques ;
- les esters de synthèse comme les huiles de formule R1COOR2 dans laquelle R1 représente le reste d'un acide gras linéaire ou ramifié comportant de 1 à 40 atomes de carbone et R2 représente une chaîne hydrocarbonée notamment ramifiée contenant de 1 à 40 atomes de carbone à condition que R1 + R2 soit 10, comme par exemple l'huile de Purcellin (octanoate de cétostéaryle), le myristate d'isopropyle, le palmitate d'isopropyle, le benzoate d'alcool en C12 à C15, le laurate d'hexyle, l'adipate de diisopropyle, l'isononanoate d'isononyle, le palmitate de 2-éthylhexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique; et leurs mélanges.

Les huiles de silicone non volatiles utilisables dans les compositions conformes à l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates.

La teneur en huile ou solvant organique non volatile dans la composition conforme à l'invention va de 0,01 à 30% en poids, en particulier de 0,1 à 25% en poids, et mieux de 0,1 à 20% par rapport au poids total de la composition.

La composition selon l'invention peut également comprendre au moins une cire. Le terme « cire » désigne une matière grasse à changement réversible liquide/solide, ayant une température de fusion supérieure à 55°C et généralement inférieure à 110°C, qui est liquide dans les conditions de préparation de la composition et qui présente une organisation cristalline anisotrope à l'état solide. La cire peut être polaire ou apolaire
Par cire polaire, on entend une cire comprenant au moins un hétéroatome tel que l'oxygène, l'azote, le silicium ou le phosphore.

En particulier, la cire polaire peut être choisie dans le groupe comprenant la cire d'abeille, la cire de carnauba, la cire de candelilla, la cire de coton, la cire de son de riz, la cire de baie, la cire d'insecte de chine, la cire de montan, la lanoline et ses dérivés alcools, acétylés, estérifiés, polyéthoxylés, la cire de kapok, la cire de canne à sucre, le laurate d'hexyle, la cire de jojoba, la cire shellac, l'éther de cholestérol polyéthoxylé, les cires d'abeille synthétiques, ou un de leurs mélanges. On peut également citer les cires d'esters végétaux tels que par exemple, à titre illustratif, les esters de jojoba, les esters d'alkyle ou d'esters d'alkyle hydrogénés.

Par cire apolaire, on entend une cire hydrocarbonée et/ou une cire siliconée.

On entend par « cire apolaire hydrocarbonée », une cire comprenant uniquement des atomes de carbone et d'hydrogène et ne comprenant pas d'hétéroatomes tels que l'oxygène, l'azote, le silicium ou le phosphore. On entend par « cire apolaire siliconée », une cire comprenant un hétéroatome de silicium.

La cire, si elle est présente dans la composition cosmétique de l'invention, représente une teneur comprise entre 0,5 à 20% en poids, de préférence de 1% à 10% en poids, par rapport au poids total de la composition.

La composition selon l'invention peut comprendre une phase aqueuse comprenant de l'eau et optionnellement au moins un solvant soluble dans l'eau. Par « solvant soluble dans l'eau », on désigne un composé liquide à température ambiante et miscible à l'eau (miscibilité dans l'eau supérieure à 50 % en poids à 25 °C et pression atmosphérique). Parmi les solvants hydrosolubles pouvant être utilisés dans les compositions conformes à l'invention, on peut citer à titre indicatif et non limitatif, les mono-alcools ayant de 1 à 5 atomes de carbone tels que l'éthanol et l'isopropanol, les cétones en C₃-C₄ et les aldéhydes en C₂-C₄.

Un ou plusieurs agent(s) actif(s), d'origine naturelle ou synthétique, ayant une activité biologique, par exemple choisis parmi les vitamines, les oligo-éléments, l'allantoïne, les protéines végétales, les extraits végétaux, les agents hydratants, les agents antiâges, les antioxydants, les agents favorisant l'éclat et des mélanges de ceux-ci. En particulier, l'agent actif est choisi parmi une eau de fruit de vanilla planifolia, le niacinamide, l'acide hyaluronique et ses dérivés, un extrait de levure et des mélanges de ceux-ci.

Les compositions conformes à l'invention peuvent également comprendre au moins une matière colorante comme les matières pulvérulentes, les colorants liposolubles, les colorants hydrosolubles.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, l'oxychlorure de bismuth, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le 13-carotène, l'huile de soja, le brun Soudan, le D&C Yellow 11, le D&C Violet 2, le D&C Orange 5, le jaune quinoléine, le rocou.

Ces matières colorantes peuvent être présentes en une teneur allant de 0,01 à 30% en poids par rapport au poids total de la composition.

Les compositions conformes à l'invention peuvent également comprendre au moins une charge.

Les charges peuvent être choisies parmi celles bien connues de l'homme du métier et couramment utilisées dans les compositions cosmétiques. Les charges peuvent être minérales ou organiques, lamellaires ou sphériques. On peut citer le mica, la silice, le kaolin, les poudres de polyamide, de poly-13-alanine et de polyéthylène, les poudres de polymères de tétrafluoroéthylène comme le Téflon ^{®} , la lauroyl-lysine, l'amidon, le nitrure de bore, les micro sphères creuses polymériques expansées telles que celles de chlorure de polyvinylidène/acrylonitrile, les poudres acryliques, les particules de polyméthacrylate de méthyle et les microbilles de résine de silicone, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, et en particulier de 12 à 18 atomes de carbone, par exemple le stéarate de zinc, de magnésium ou de lithium, le laurate de zinc, le myristate de magnésium.

On peut également utiliser un composé susceptible de gonfler à la chaleur et notamment des particules thermoexpansibles telles que les microsphères non expansées de copolymère de chlorure de vinylidène/d'acrylonitrile/méthacrylate de méthyle ou de copolymère d'homopolymère d'acrylonitrile.

Les charges peuvent représenter de 0,1 à 25% en particulier de 0,2 à 20% en poids par rapport au poids total de la composition.

Les compositions conformes à l'invention peuvent également comprendre au moins une fibre.

Par "fibre", il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur section peut être quelconque et notamment de section circulaire ou polygonale (carrée, hexagonale ou octogonale) selon l'application spécifique envisagée. Leur forme peut être linéaire, courbe, sinusoïdale ou frisée. En particulier, leurs extrémités sont épointées et/ou polies pour éviter de se blesser.

A titre de fibres utilisables dans la composition selon l'invention, on peut citer les fibres non rigides telles que les fibres de polyamide (Nylon^{®}) ou les fibres rigides telles que les fibres de polyimide-amide ou de poly-(p-phénylènetéréphtalamide) (ou d'aramide).

Les fibres peuvent être présentes dans la composition selon l'invention en une teneur allant de 0,01% à 10% en poids, par rapport au poids total de la composition, en particulier de 0,1% à 5% en poids, et plus particulièrement de 0,3% à 3% en poids

D'autres additifs habituellement utilisés en cosmétique peuvent également être présents dans la composition selon l'invention, notamment des conservateurs, des agents antioxydants, des filtres UV organiques et/ou inorganiques, ou des parfums bien connus dans le domaine technique.

L'homme du métier est en mesure de choisir, parmi l'ensemble de ces éventuels additifs, aussi bien la nature que la quantité de ceux qui seront ajoutés à la composition, de telle sorte que celle-ci conserve l'ensemble de ses propriétés. Bien entendu, l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

La composition cosmétique peut être à destination d'un produit cosmétique choisi notamment parmi les rouges à lèvres, les gloss, les fonds de teint fluide, compact, les vernis à ongles, les ombres à paupières, les mascaras, les crayons à cils ou à sourcils, les crèmes de soins, les sérums, Is produits à usage de protection solaire, les toiletries telles que les gels douches, etc...

Le biopolymère peut être obtenu de façon connue par biosynthèse avec le microorganisme enregistré à la Collection Nationale de Culture des Microorganismes (Institut Pasteur, 25-28, Rue du Dr. Roux, 75724 Paris Cédex 15, France) sous le traité de Budapest sous le numéro CNCM I-5658, dépôt effectué au nom de la société Polymaris Biotechnology, sise à Brest - France. La présente invention ne couvre donc pas expressément ces souches mais seulement l'utilisation des polyhydroxyalcanoates secrétés par celles-ci pour la réalisation de composition cosmétiques.

Le polyhydroxyalcanoate mis en oeuvre dans le cadre des compositions cosmétiques selon l'invention peut être obtenu par fermentation de la bactérie enregistrée en vertu du traité de Budapest à la Collection Nationale de Cultures de Microorganismes le 24 février 2021 sous le numéro de dépôt I-5658 dans un milieu de culture approprié, conventionnellement sous agitation et aéré. La fermentation pour produire le polyhydroxyalcanoate est réalisée dans des conditions d'agitation et d'aération du milieu à une température comprise entre 20°C et 37°C, préférentiellement à 30°C, le milieu ayant un pH compris entre 6,5 et 9 préférentiellement proche de 7,5, et étant ajusté si nécessaire durant la fermentation. La durée de la fermentation est comprise entre 24 et 120 heures, préférentiellement comprise entre 36 et 72 heures. Selon un mode de réalisation de l'invention, des sels minéraux sont apportés durant la fermentation de la bactérie. A titre illustratif et non limitatif, on peut citer parmi les sels minéraux ceux aptes à fournir au milieu de culture des ions Na⁺, K⁺, NH₄⁺, Ca²⁺, Mg²⁺, PO₄³⁻, SO₄²⁻, Cl⁻, CO₃⁻ ou des éléments essentiels tels que Cu, Mn, Fe et du Zn.

Les méthodes d'isolation et de purification du polyhydroxyalcanoate sont obtenues par des moyens classiques et bien connus de l'homme de l'art, tels que la centrifugation, la filtration, l'ultrafiltration, l'extraction, l'évaporation et la dialyse.

Dans un mode préféré de l'invention, le copolymère de polyhydroxyalcanoate produit par la bactérie enregistrée sous le numéro CNCM I-5658 est caractérisée par la présence d'unités polymériques consistant en :
- 3% en mole d'unités monomériques (A1),
- 3% en mole d'unités monomériques (B1),
- 25% en mole d'unités monomériques (C1)
- 13% en mole d'unités monomériques (D1)
- 45% en mole d'unités monomériques (E1)
- 11% en mole d'unités monomériques (F1)

L'invention sera maintenant illustrée par les exemples non limitatifs suivants.

### Exemples

### Exemple 1 : préparation et isolation du polyhydroxyalcanoate produit par la bactérie enregistrée sous le numéro de dépôt CNCM I-5658

### a) Méthode de culture de la bactérie enregistrée sous le numéro de dépôt CNCM I-5658

Pour la production du biopolymère, l'ensemble des opérations sont réalisées selon les bonnes pratiques de microbiologie en conditions stériles. Les différents milieux sont également stérilisés soit par traitement thermique (121°C pendant 20 minutes) ou par filtration 0,2µm.

Un cryotube de 2 mL de la souche CNCM I-5658 est inoculé dans un erlenmeyer contenant 50 mL de milieu permettant la culture de microorganismes marin (milieu marine broth E2216, Difco, France). La culture est incubée à 30°C sous agitation pendant 12 à 18 heures.

La culture obtenue est ensuite inoculée dans un erlenmeyer contenant 1,45 L de milieu minéral adapté à sa croissance (milieu M1 - Tableau 1) à 30°C sous agitation pendant 8 heures.

Enfin, la culture est inoculée en fermenteur contenant 13,5 L de milieu minéral de culture adaptée à sa croissance et la biosynthèse du biopolymère (milieu M2 - Tableau 2). La culture est maintenue à 30°C sous agitation pendant au minimum 30 heures.

Pendant les 28 premières heures de culture, il est ajouté toutes une heure et 30 minutes, 20 mL d'une solution d'acide décanoïque et d'acide undécanoïque (dans un ratio 70/30) tamponnée à pH 6 avec du NaOH 10N.

Le pH est régulé à 7,2 par du NaOH 10 N ou du H₂SO₄ 2N.

L'oxygénation et l'agitation de la culture sont également régulés pour permettre la croissance des cellules et l'accumulation du biopolymère.

A la fin de la fermentation, la biomasse cellulaire contenant les biopolymères est concentrée par centrifugation.

Cette biomasse est ensuite séchée afin d'éliminer toute l'eau et jusqu'à obtention d'une masse constante.

**[Tableau 1] - Composition du milieu M1**

| Éléments | g/Kg dans H₂O | g/Kg dans H₂O |
|---|---|---|
| NaNH₄HPO₄4H₂O | 3,5 | |
| KH₂PO₄ | 3,7 | |
| K₂HPO4 | 7,5 | |
| MgSO4 | 0,25 | |
| NaCl | 20 | |
| **Solution n° 1A** | 40 | |
| Citrate de sodium | | 250 |
| Extrait de levure | | 25 |
| **Solution n°2** | 1,3 | |
| FeSO₄.7H₂O | | 2,78 |
| CaCl₂.2H₂O | | 1,47 |
| MnCl₂.4 H₂O | | 1,98 |
| CoCl₂.6 H₂O | | 2,38 |
| ZnSO₄.7 H₂O | | 0,29 |
| CuCl₂.2 H₂O | | 0,17 |

**[Tableau 2] - Composition du milieu M2**

| Éléments | g/Kg dans H2O | g/Kg dans H2O |
|---|---|---|
| NaNH4HPO4 4H2O | 3,5 | |
| KH2PO4 | 3,7 | |
| K2HPO4 | 7,5 | |
| MgSO4 | 0,25 | |
| NaCl | 20 | |
| Solution n° 1B | 50 | |
| Citrate de sodium | | 200 |
| Extrait de levure | | 2 |
| Solution n°2 | 1,0 | |
| FeSO4.7H2O | | 2,78 |
| CaCl2.2H2O | | 1,47 |
| MnCl2.4 H2O | | 1,98 |
| CoCl2.6 H2O | | 2,38 |
| ZnSO4.7 H2O | | 0,29 |
| CuCl2.2 H2O | | 0,17 |

### b) Purification du biopolymère de polyhydroxyalcanoate

L'extraction du biopolymère polyhydroxyalcanoate est réalisée par ajout de dichlorométhane à raison de 10 % de biomasse sèche par volume de solvant à température ambiante et sous agitation pendant 18 heures.

La biomasse est éliminée par centrifugation (8000 rpm X 20 min) et le surnageant contenant le biopolymère est récupéré.

Ce dernier peut être filtré afin d'améliorer la qualité du biopolymère final.

Il est concentré par élimination du dichlorométhane à l'aide d'un évaporateur rotatif.

Le biopolymère concentré en solution dans le dichlorométhane est enfin précipité par ajout d'une solution d'éthanol à froid (10 :1) puis séché dans une étuve ventilée à 40°C jusqu'à évaporation complète du solvant.

### Exemple 2: caractérisation physico-chimique des biopolymère polyhydroxyalcanoate obtenu dans l'exemple 1

Le poids moléculaire est déterminé par chromatographie d'exclusion stérique avec pour détection un réfractomètre (RI).

Le poids moléculaire est déterminé par comparaison du temps de rétention du polymère avec le temps de rétention de standards Polystyrene via une droite d'étalonnage.

L'analyse permet de déterminer le poids moléculaire moyen (Mw en Dalton), le poids moléculaire moyen en nombre (Mn en Dalton) en équivalent PS (polystyrène), ainsi que l'indice de polydispersité.

La composition du biopolymère est déterminée par chromatographie en phase gazeuse munie d'un détecteur à ionisation de flamme (FID) et confirmée par analyse RMN 1H, 13C, HMBC et HMQC.

On a ainsi pu isoler un copolymère 1 consistant en 3% en poids de poly(hydroxy-3-hexanoate), 3% en poids de poly(hydroxy-3-heptanoate), 25% en poids de poly(hydroxy-3-octanoate), 13% en poids de poly(hydroxy-3-nonanoate), 45% en poids de poly(hydroxy-3-decanoate), et 11% en poids de poly(hydroxy-3-undecanoate).

On a ainsi également isolé un copolymère 2 consistant en 2% en poids de poly(hydroxy-3-hexanoate), 6% en poids de poly(hydroxy-3-heptanoate), 21% en poids de poly(hydroxy-3-octanoate), 24% en poids de poly(hydroxy-3-nonanoate), 30% en poids de poly(hydroxy-3-decanoate), et 17% en poids de poly(hydroxy-3-undecanoate).

On a enfin pu isoler un copolymère 3 consistant en 1% en poids de poly(hydroxy-3-hexanoate), 5% en poids de poly(hydroxy-3-heptanoate), 22% en poids de poly(hydroxy-3-octanoate), 15% en poids de poly(hydroxy-3-nonanoate), 43% en poids de poly(hydroxy-3-decanoate), et 14% en poids de poly(hydroxy-3-undecanoate).

### Exemple 3 : Evaluation des propriétés filmogènes du copolymère 1 de l'exemple 2

Dans une première étape, on prépare une solution du polymère dont on souhaite évaluer les propriétés filmogènes dans un solvant volatile apte à le solubiliser. Le polymère représente 30 à 50% en masse de la solution. Dans une seconde étape on dépose une solution obtenue à l'étape 1 dans le fond d'une boîte de Pétri. Le choix de la boîte de Pétri permet de se rapprocher au plus près des conditions réelles d'utilisation d'une composition cosmétique, le matériau constitutif de la boite de Pétri présentant une énergie de surface similaire à celle de la peau humaine. Ainsi la peau non traitée présente approximativement une énergie de surface de 38 mN/m et une peau lavée au savon, une énergie de surface de l'ordre de 25 à 29 mN/m (H. Schott, Contact angles and wettability of human skin, J. Pharm Sci. 60 (1971) 1893-1895), une boîte de Pétri plastique en matériau classique polystryrène présente quant à elle une énergie de surface de l'ordre de 39 mN/m. Dans une troisième étape, on place la boîte de Pétri dans une étuve ventilée à 45°C afin d'évaporer le solvant volatile, et obtenir un dépôt de film sec d'environ 1g, puis on caractérise la qualité du film de polymère déposé au fond de la boîte de Pétri selon 3 critères d'appréciation:
- la transparence du film : une note de 1 est attribuée à un film qualifié de transparent (pas de déformation de l'image et de son contraste par son observation au travers du film), 0 pour un film opaque ;
- la régularité du film : une note de 0, 1, ou 2 est attribuée, la note de 2 caractérisant un dépôt régulier en épaisseur et couvrant l'ensemble de la surface, et la note de 0 caractérisant un film fortement craquelé ou présentant des agrégats ;
- l'adhésion du film : une note de 4 à 0 est attribuée sur la base des caractéristiques suivantes ; 4 - très adhésif, ne se décolle pas dans les 48h ; 3 - adhésif, se décolle partiellement sous la contrainte ; 2 - adhésif initialement, se décolle partiellement dans les 48h ; 1 - faible adhésif, se décolle totalement dans les 48h ; 0 - pas d'adhésion initial.

Plus la note du polymère testé est haute, meilleure sont ses qualités filmogènes :
Le copolymère 1 obtenu dans l'exemple 2 est ainsi évalué et ses propriétés filmogènes sont comparées à celles obtenues selon le même protocole pour le Belsil TMS 803 (nom INCI : Trimethyl silicate) commercialisé par la société AES, un filmogène de l'état de l'art couramment utilisé dans le domaine cosmétique. Les résultats sont présentés dans le Tableau 3.

**[Tableau 3] - Evaluation des propriétés filmogènes :**

| Polymère évalué | Transparence | Régularité | Adhésion |
|---|---|---|---|
| Belsil TMS 803 | 1 | 2 | 4 |
| Exemple 2 selon l'invention | 1 | 2 | 4 |

On constate que le copolymère 1 de l'exemple 2 obtenu par un procédé de biosynthèse, propre et respectueux de l'environnement présente des critères d'évaluation similaire au polymère de l'art antérieur. Il est donc un excellent candidat pour substituer les polymères filmogènes synthétiques de l'art antérieur, et permettre ainsi d'obtenir des compositions cosmétiques présentant le même niveau de qualité et de performance, tout en étant plus respectueux de l'environnement avec notamment une excellente biodégradabilité.

### Exemple 4 : Composition cosmétique de base de teint

On a préparé une base de teint sous forme d'émulsion eau-dans-huile ayant la composition présentée dans le tableau 4 suivant :

**[Tableau 4]**

| Nom INCI | Teneur (% en poids) |
|---|---|
| Copolymère 1 de l'exemple 2 | 8 |
| GLYCERINE | 10 |
| SORBITOL & WATER (70% MA) | 3,5 |
| PENTYLENE GLYCOL | 2,5 |
| POLYGLYCERYL-3 DIISOSTEARATE (CITHROL PG32IS-LQ) | 3,0 |
| DIMETHICONE & DIMETHICONE/PEG-10/15 CROSSPOLYMER (KSG-210) | 10 |
| UNDECANE & TRIDECANE & TOCOPHEROL & HELIANTHUS ANNUUS (SUNFLOWER) SEED OIL (CETIOL ULTIMATE) | 12 |
| EAU DEMINERALISEE | 50,5 |
| CONSERVATEUR | 0,5 |

On a préparé la base de teint selon le protocole suivant :
1-peser la phase aqueuse comprenant l'eau, la glycérine, le sorbitol, le pentylène glycol et le conservateur et la mettre sous agitation Rayneri,
2- ajouter le copolymère 1 de l'Exemple 2 sous agitation 10 min à 250 rpm jusqu'à formation du gel,
3- mélanger le CITHROL, le KSG-210 et le CETIOL ULTIMATE sous agitation dans un rotor stator à 245 rpm pendant 5min,
4- ajouter les pigments sous agitation dans un rotor stator à 350 rpm dans la phase grasse - introduire la phase aqueuse (1+2) dans ce mélange (3+4) dans un rotor stator à 245 rpm pendant 5min pour former l'émulsion.

La base de teint obtenue est fluide et homogène, elle s'applique facilement sans pelucher. Le film formé sur la peau est uniforme.

### Référence à du matériel biologique déposé

Dans la présente demande, il est fait référence à (aux) matériel(s) biologique(s) suivant(s) et aux souches variantes ou mutantes dérivées :
- référence d'identification « 939 » et numéro d'enregistrement « I-5658 » déposée à la Collection Nationale de Cultures de Micro-organismes (CNCM), en France, le 24 février 2021, par Polymaris Biotechnology dont l'adresse est 160 rue Pierre Rivoalon, 29200 Brest, France.

## Revendications

1. Composition cosmétique comprenant au moins un copolymère de polyhydroxyalcanoate, **caractérisée en ce que** ledit copolymère de polyhydroxyalcanoate consiste en une répétition d'unités monomériques de formules (A1), (B1), (C1), (D1), (E1), et (F1) suivantes :
Formule (A1) : -[-O-CH(R1)-(CH2)-CO-]-
Formule (B1) : -[-O-CH(R2)-(CH2)-CO-]-
Formule (C1) : -[-O-CH(R3)-(CH2)-CO-]-
Formule (D1) : -[-O-CH(R4)-(CH2)-CO-]-
Formule (E1) : -[-O-CH(R5)-(CH2)-CO-]-
Formule (F1) : -[-O-CH(R6)-(CH2)-CO-]-
dans lesquelles :
R1 représente un groupe n-propyle
R2 représente un groupe n-butyle
R3 représente un groupe n-pentyle
R4 représente un groupe n-hexyle
R5 représente un groupe n-heptyle
R6 représente un groupe n-octyle
ledit copolymère de polyhydroxyalcanoate comprenant au moins 1% en moles et au plus 50% en moles de chacune des d'unités monomériques de formule (A1), (B1), (C1), (D1), (E1), et (F1).

2. Composition cosmétique selon la revendication précédente, **caractérisée en ce que** ledit copolymère de polyhydroxyalcanoate consiste en :
- 1% à 3% en mole d'unités monomériques (A1),
- 2 à 9% en mole d'unités monomériques (B1),
- 20 à 30% en mole d'unités monomériques (C1),
- 10 à 30% en mole d'unités monomériques (D1),
- 25 à 50% en mole d'unités monomériques (E1), et
- 9 à 20% en mole d'unités monomériques (F1).

3. Composition cosmétique selon l'une des revendications précédentes, **caractérisée en ce que** ledit copolymère de polyhydroxyalcanoate consiste en :
- 1% à 3% en mole d'unités monomériques (A1),
- 3% à 7% en mole d'unités monomériques (B1),
- 20% à 25% en mole d'unités monomériques (C1),
- 13% à 25% en mole d'unités monomériques (D1),
- 30 à 45% en mole d'unités monomériques (E1), et
- 10% à 18% en mole d'unités monomériques (F1).

4. Composition cosmétique selon l'une des revendications précédentes, **caractérisée en ce que** ledit copolymère de polyhydroxyalcanoate consiste en :
- 3% en mole d'unités monomériques (A1),
- 3% en mole d'unités monomériques (B1),
- 25% en mole d'unités monomériques (C1),
- 13% en mole d'unités monomériques (D1),
- 45% en mole d'unités monomériques (E1), et
- 11% en mole d'unités monomériques (F1).

5. Composition cosmétique selon l'une des revendications précédentes, **caractérisée en ce que** ledit copolymère de polyhydroxyalcanoate présente un poids moléculaire moyen (Mw) compris entre 20 000 à 100 000 g/mol.

6. Composition cosmétique selon l'une des revendications précédentes, **caractérisée en ce que** ledit copolymère de polyhydroxyalcanoate est présent en une teneur comprise entre 0,5 % à 30% en poids, de préférence 1% à 20% en poids, et très préférentiellement 2% à 8% en poids, par rapport au poids total de la composition.

7. Composition cosmétique selon l'une des revendications précédentes **caractérisée en ce que** ledit copolymère de polyhydroxyalcanoate est obtenu selon un procédé de fermentation en présence d'une bactérie enregistrée en vertu du traité de Budapest à la Collection Nationale de Cultures de Microorganismes le 24 février 2021 sous le numéro de dépôt I-5658.

8. Composition cosmétique selon l'une des revendications précédentes, **caractérisée en ce qu'**elle se présente sous la forme d'une émulsion, d'une microémulsion, d'une nanoémulsion, d'une poudre, d'une composition anhydre, d'une suspension, d'une lotion, d'une crème, d'un gel aqueux ou hydroalcoolique, d'une mousse, d'un sérum, d'une solution ou d'une dispersion pour aérosol, ou d'une dispersion de vésicules lipidiques, ou de gouttelettes obtenues par coacervation ou par microfluidique.

9. Composition cosmétique selon l'une des revendications précédentes, **caractérisée en ce qu'**elle comprend au moins un composé choisi parmi un agent émollient ou humectant, un agent gélifiant et/ou épaississant, un agent tensioactif, une huile, une cire, un colorant, un pigment, une nacre, une charge, un conservateur, un agent antioxydant, un agent actif, une poudre organique ou inorganique, un solvant volatile et/ou non volatile, un solubilisant, un chélatant, un séquestrant, un sel ionique, un filtre solaire et un parfum.

10. Utilisation d'au moins un copolymère de polyhydroxyalcanoate en tant qu'agent filmogène dans une composition cosmétique, ledit copolymère de polyhydroxyalcanoate consistant en une répétition d'unités monomériques de formules (A1), (B1), (C1), (D1), (E1), et (F1) suivantes :
Formule (A1) : -[-O-CH(R1)-(CH2)-CO-]-
Formule (B1) : -[-O-CH(R2)-(CH2)-CO-]-
Formule (C1) : -[-O-CH(R3)-(CH2)-CO-]-
Formule (D1) : -[-O-CH(R4)-(CH2)-CO-]-
Formule (E1) : -[-O-CH(R5)-(CH2)-CO-]-
Formule (F1) : -[-O-CH(R6)-(CH2)-CO-]-
dans lesquelles :
R1 représente un groupe n-propyle
R2 représente un groupe n-butyle
R3 représente un groupe n-pentyle
R4 représente un groupe n-hexyle
R5 représente un groupe n-heptyle
R6 représente un groupe n-octyle
ledit copolymère de polyhydroxyalcanoate comprenant au moins 1% en moles et au plus 50% en moles de chacune des d'unités monomériques de formule (A1), (B1), (C1), (D1), (E1), et (F1) ;
ledit copolymère de polyhydroxyalcanoate étant obtenu par un procédé de fermentation à partir d'une bactérie enregistrée en vertu du traité de Budapest à la Collection Nationale de Cultures de Microorganismes le 24 février 2021 sous le numéro de dépôt I-5658 ; et
étant présent en une teneur comprise entre 2 et 20% en poids, par rapport au poids total de ladite composition cosmétique..

## Claims

1. Cosmetic composition comprising at least one polyhydroxyalkanoate copolymer, **characterized in that** said polyhydroxyalkanoate copolymer consists of a repetition of monomer units of following formulae (A1), (B1), (C1), (D1), (E1) and (F1):
formula (A1): -[-O-CH(R1)-(CH₂)-CO-]-
formula (B1) : -[-O-CH(R2)-(CH₂)-CO-]-
formula (C1) : -[-O-CH(R3)-(CH₂)-CO-]-
formula (D1) : -[-O-CH(R4)-(CH₂)-CO-]-
formula (E1) : -[-O-CH(R5)-(CH₂)-CO-]-
formula (F1) : -[-O-CH(R6)-(CH₂)-CO-]-
in which:
R1 represents an n-propyl group
R2 represents an n-butyl group
R3 represents an n-pentyl group
R4 represents an n-hexyl group
R5 represents an n-heptyl group
R6 represents an n-octyl group
said polyhydroxyalkanoate copolymer comprising at least 1 mol% and at most 50 mol% of each of the monomer units of formulae (A1), (B1), (C1), (D1), (E1) and (F1).

2. Cosmetic composition according to the preceding claim, **characterized in that** said polyhydroxyalkanoate copolymer consists of:
- from 1 mol% to 3 mol% of monomer units (A1),
- from 2 mol% to 9 mol% of monomer units (B1),
- from 20 mol% to 30 mol% of monomer units (C1),
- from 10 mol% to 30 mol% of monomer units (D1),
- from 25 mol% to 50 mol% of monomer units (E1), and
- from 9 mol% to 20 mol% of monomer units (F1).

3. Cosmetic composition according to either of the preceding claims, **characterized in that** said polyhydroxyalkanoate copolymer consists of:
- from 1 mol% to 3 mol% of monomer units (A1),
- from 3 mol% to 7 mol% of monomer units (B1),
- from 20 mol% to 25 mol% of monomer units (C1),
- from 13 mol% to 25 mol% of monomer units (D1),
- from 30 mol% to 45 mol% of monomer units (E1), and
- from 10 mol% to 18 mol% of monomer units (F1).

4. Cosmetic composition according to one of the preceding claims, **characterized in that** said polyhydroxyalkanoate copolymer consists of:
- 3 mol% of monomer units (A1),
- 3 mol% of monomer units (B1),
- 25 mol% of monomer units (C1),
- 13 mol% of monomer units (D1),
- 45 mol% of monomer units (E1), and
- 11 mol% of monomer units (F1).

5. Cosmetic composition according to one of the preceding claims, **characterized in that** said polyhydroxyalkanoate copolymer exhibits an average molecular weight (Mw) of between 20 000 and 100 000 g/mol.

6. Cosmetic composition according to one of the preceding claims, **characterized in that** said polyhydroxyalkanoate copolymer is present in a content of between 0.5% and 30% by weight, preferably from 1% to 20% by weight and very preferentially from 2% to 8% by weight, with respect to the total weight of the composition.

7. Cosmetic composition according to one of the preceding claims, **characterized in that** said polyhydroxyalkanoate copolymer is obtained according to a fermentation process in the presence of a bacterium registered under the Treaty of Budapest at the Collection Nationale de Cultures de Microorganismes [French National Collection of Microorganism Cultures] on 24 February 2021 under the deposit number I-5658.

8. Cosmetic composition according to one of the preceding claims, **characterized in that** it is provided in the form of an emulsion, of a microemulsion, of a nanoemulsion, of a powder, of an anhydrous composition, of a suspension, of a lotion, of a cream, of an aqueous or aqueous-alcoholic gel, of a foam, of a serum, of an aerosol solution or dispersion, or of a dispersion of lipid vesicles, or of droplets obtained by coacervation or by microfluidics.

9. Cosmetic composition according to one of the preceding claims, **characterized in that** it comprises at least one compound chosen from an emollient or humectant agent, a gelling and/or thickening agent, a surface-active agent, an oil, a wax, a dye, a pigment, a pearlescent agent, a filler, a preservative, an antioxidant, an active agent, an organic or inorganic powder, a volatile and/or non-volatile solvent, a solubilizing agent, a chelating agent, a sequestering agent, an ionic salt, a sunscreen and a fragrance.

10. Use of at least one polyhydroxyalkanoate copolymer as film-forming agent in a cosmetic composition, said polyhydroxyalkanoate copolymer consisting of a repetition of monomer units of following formulae (A1), (B1), (C1), (D1), (E1) and (F1):
formula (A1) : -[-O-CH(R1)-(CH₂)-CO-]-
formula (B1) : -[-O-CH(R2)-(CH₂)-CO-]-
formula (C1): -[-O-CH(R3)-(CH₂)-CO-]-
formula (D1) : -[-O-CH(R4)-(CH₂)-CO-]-
formula (E1) : -[-O-CH(R5)-(CH₂)-CO-]-
formula (F1): -[-O-CH(R6)-(CH₂)-CO-]-
in which:
R1 represents an n-propyl group
R2 represents an n-butyl group
R3 represents an n-pentyl group
R4 represents an n-hexyl group
R5 represents an n-heptyl group
R6 represents an n-octyl group
said polyhydroxyalkanoate copolymer comprising at least 1 mol% and at most 50 mol% of each of the monomer units of formulae (A1), (B1), (C1), (D1), (E1) and (F1);
said polyhydroxyalkanoate copolymer being obtained by a fermentation process starting from a bacterium registered under the Treaty of Budapest at the Collection Nationale de Cultures de Microorganismes [French National Collection of Microorganism Cultures] on 24 February 2021 under the deposit number I-5658; and
being present in a content of between 2% and 20% by weight, with respect to the total weight of said cosmetic composition.

## Patentansprüche

1. Kosmetikzusammensetzung, umfassend mindestens ein Polyhydroxyalkanoat-Copolymer, **dadurch gekennzeichnet, dass** das Polyhydroxyalkanoat-Copolymer aus einer Wiederholung von Monomereinheiten der Formeln (A1), (B1), (C1), (D1), (E1) und (F1) besteht:
Formel (A1): -[-O-CH(R1)-(CH2)-CO-]-
Formel (B1): -[-O-CH(R2)-(CH2)-CO-]-
Formel (C1): -[-O-CH(R3)-(CH2)-CO-]-
Formel (D1): -[-O-CH(R4)-(CH2)-CO-]-
Formel (E1): -[-O-CH(R5)-(CH2)-CO-]-
Formel (F1): -[-O-CH(R6)-(CH2)-CO-]-,
worin:
R1 eine n-Propylgruppe darstellt
R2 eine n-Butylgruppe darstellt
R3 eine n-Pentylgruppe darstellt
R4 eine n-Hexylgruppe darstellt
R5 eine n-Heptylgruppe darstellt
R6 eine n-Octylgruppe darstellt,
wobei das Polyhydroxyalkanoat-Copolymer mindestens 1 mol-% und höchstens 50 mol-% jeder der Monomereinheiten der Formel (A1), (B1), (C1), (D1), (E1) und (F1) umfasst.

2. Kosmetikzusammensetzung nach dem vorhergehenden Anspruch, **dadurch gekennzeichnet, dass** das Polyhydroxyalkanoat-Copolymer aus Folgendem besteht:
- 1 mol-% bis 3 mol-% Monomereinheiten (A1),
- 2 bis 9 mol-% Monomereinheiten (B1),
- 20 bis 30 mol-% Monomereinheiten (C1),
- 10 bis 30 mol-% Monomereinheiten (D1),
- 25 bis 50 mol-% Monomereinheiten (E1) und
- 9 bis 20 mol-% Monomereinheiten (F1).

3. Kosmetikzusammensetzung nach einem der vorhergehenden Ansprüche, **dadurch gekennzeichnet, dass** das Polyhydroxyalkanoat-Copolymer aus Folgendem besteht:
- 1 mol-% bis 3 mol-% Monomereinheiten (A1),
- 3 mol-% bis 7 mol-% Monomereinheiten (B1),
- 20 mol-% bis 25 mol-% Monomereinheiten (C1),
- 13 mol-% bis 25 mol-% Monomereinheiten (D1),
- 30 bis 45 mol-% Monomereinheiten (E1) und
- 10 mol-% bis 18 mol-% Monomereinheiten (F1).

4. Kosmetikzusammensetzung nach einem der vorhergehenden Ansprüche, **dadurch gekennzeichnet, dass** das Polyhydroxyalkanoat-Copolymer aus Folgendem besteht:
- 3 mol-% Monomereinheiten (A1),
- 3 mol-% Monomereinheiten (B1),
- 25 mol-% Monomereinheiten (C1),
- 13 mol-% Monomereinheiten (D1),
- 45 mol-% Monomereinheiten (E1) und
- 11 mol-% Monomereinheiten (F1).

5. Kosmetikzusammensetzung nach einem der vorhergehenden Ansprüche, **dadurch gekennzeichnet, dass** das Polyhydroxyalkanoat-Copolymer ein mittleres Molekulargewicht (Mw) zwischen 20.000 bis 100.000 g/mol aufweist.

6. Kosmetikzusammensetzung nach einem der vorhergehenden Ansprüche, **dadurch gekennzeichnet, dass** das Polyhydroxyalkanoat-Copolymer in einem Gehalt zwischen 0,5 Gew.-% bis 30 Gew.-%, vorzugsweise 1 Gew.-% bis 20 Gew.-% und sehr stark bevorzugt 2 Gew.-% bis 8 Gew.-% bezogen auf das Gesamtgewicht der Zusammensetzung vorliegt.

7. Kosmetikzusammensetzung nach einem der vorhergehenden Ansprüche, **dadurch gekennzeichnet, dass** das Polyhydroxyalkanoat-Copolymer gemäß einem Fermentationsverfahren in Gegenwart eines Bakteriums erhalten wird, das nach dem Budapester Vertrag am 24. Februar 2021 bei der Collection Nationale de Cultures de Microorganismes unter der Hinterlegungsnummer 1-5658 registriert wurde.

8. Kosmetikzusammensetzung nach einem der vorhergehenden Ansprüche, **dadurch gekennzeichnet, dass** sie in Form einer Emulsion, einer Mikroemulsion, einer Nanoemulsion, eines Pulvers, einer wasserfreien Zusammensetzung, einer Suspension, einer Lotion, einer Creme, eines wässrigen oder wässrig-alkoholischen Gels, eines Schaums, eines Serums, einer Aerosollösung oder - dispersion oder einer Dispersion von Lipidvesikeln oder von durch Koazervation oder Mikrofluidik gewonnenen Tröpfchen vorliegt.

9. Kosmetikzusammensetzung nach einem der vorhergehenden Ansprüche, **dadurch gekennzeichnet, dass** sie mindestens eine Verbindung umfasst, die aus einem Weichmacher- oder Feuchthaltemittel, einem Gelbildungs- und/oder Verdickungsmittel, einem Tensid, einem Öl, einem Wachs, einem Färbemittel, einem Pigment, einem Perlmutt, einem Füllstoff, einem Konservierungsmittel, einem Antioxidans, einem Wirkstoff, einem organischen oder anorganischen Pulver, einem flüchtigen und/oder nicht flüchtigen Lösungsmittel, einem Lösungsvermittler, einem Chelatbildner, einem Sequestrierungsmittel, einem ionischen Salz, einem Sonnenschutzfilter und einem Parfüm ausgewählt ist.

10. Verwendung mindestens eines Polyhydroxyalkanoat-Copolymers als Filmbildner in einer Kosmetikzusammensetzung, wobei das Polyhydroxyalkanoat-Copolymer aus einer Wiederholung von Monomereinheiten der Formeln (A1), (B1), (C1), (D1), (E1) und (F1) besteht:
Formel (A1): -[-O-CH(R1)-(CH2)-CO-]-,
Formel (B1): -[-O-CH(R2)-(CH2)-CO-]-,
Formel (C1): -[-O-CH(R3)-(CH2)-CO-]-,
Formel (D1): -[-O-CH(R4)-(CH2)-CO-]-,
Formel (E1): -[-O-CH(R5)-(CH2)-CO-]-,
Formel (F1): -[-O-CH(R6)-(CH2)-CO-]-,
worin:
R1 eine n-Propylgruppe darstellt
R2 eine n-Butylgruppe darstellt
R3 eine n-Pentylgruppe darstellt
R4 eine n-Hexylgruppe darstellt
R5 eine n-Heptylgruppe darstellt
R6 eine n-Octylgruppe darstellt,
wobei das Polyhydroxyalkanoat-Copolymer mindestens 1 mol-% und höchstens 50 mol-% jeder der Monomereinheiten der Formel (A1), (B1), (C1), (D1), (E1) und (F1) umfasst,
wobei das Polyhydroxyalkanoat-Copolymer durch ein Fermentationsverfahren ausgehend von einem Bakterium erhalten wird, das nach dem Budapester Vertrag am 24.
Februar 2021 bei der Collection Nationale de Cultures de Microorganismes unter der Hinterlegungsnummer 1-5658 registriert wurde, und
in einer Menge zwischen 2 und 20 Gew.-% bezogen auf das Gesamtgewicht der Kosmetikzusammensetzung vorliegt.
